# EUROPEAN PATENT APPLICATION

(11) **EP 3 162 892 A1**
(43) Date of publication of application: **03.05.2017**
(21) Application number: 15811669.9
(22) Date of filing: 24.06.2015
(51) Int. Cl.: C12N 5/071, C12M 3/00, C12N 5/10, C12P 21/00

(54) **METHOD FOR CULTURING ADHESIVE CELLS, CULTURE VESSEL, AND METHOD FOR PRODUCING PROTEIN**

(30) Priority: 26.06.2014 JP 2014130944
(71) Applicant: Zeon Corporation, Tokyo 100-8246 (JP)
(72) Inventor: ICHIMURA, Naoya, Tokyo 100-8246 (JP); HIRANO, Takaaki, Tokyo 100-8246 (JP)
(74) Representative: Vossius & Partner Patentanwälte Rechtsanwälte mbB
(86) International application number: PCT/JP2015/068155
(87) International publication number: WO 2015/199119

(57) **Abstract**

The present invention is a method for culturing adherent cells that can allow adherent cells to survive and grow even in a suspended state, a culture vessel that is formed using an alicyclic structure-containing polymer, and includes a liquid medium, and adherent cells that survive in the liquid medium in a suspended state, and a method for producing a protein. The adhesive cells can be grown in a method for culturing adherent cells comprising bringing adherent cells into contact with an alicyclic structure-containing polymer formed article to grow the adherent cells in a state in which the adherent cells are suspended in a liquid medium.

## Description

### TECHNICAL FIELD

The present invention relates to a method for culturing adherent cells that can allow adherent cells to survive and grow even in a suspended state, a culture vessel that is formed using an alicyclic structure-containing polymer, and includes a liquid medium, and adherent cells that survive in the liquid medium in a suspended state, and a method for producing a protein.

### BACKGROUND ART

A technique that produces the desired protein using a genetic recombination method is employed for drug discovery research as well as research with regard to pluripotent cells and stem cells. For example, recombinant drugs that include a protein (e.g., an antibody used for cancer treatment or rheumatism treatment, or erythropoietin (EPO) (i.e., a hormone that increases the production of red blood cells)) as a component are known. The recombinant drugs are considered to be less risky than drugs produced by known chemical synthesis as to the occurrence of unexpected side effects, and have attracted much attention.

However, since the recombinant drugs must be administered in high doses when used for treatment, and require high production cost, the recombinant drugs have not been used widely. Therefore, it has been desired to produce the recombinant drugs with improved productivity.

For example, the recombinant drugs are produced by transferring the gene of the desired protein into CHO cells (i.e., adherent cells), acclimating the CHO cells to a serum-free medium, and effecting the biosynthesis of the desired protein in the recombinant CHO cells that can grow in a suspended state. It is preferable to culture the cells at a high cell density in order to improve the productivity of the recombinant drugs.

A method that improves the productivity of the desired protein using a microcarrier is known (see Non-Patent Literature 1). Specifically, CHO cells are caused to adhere to a microcarrier, and dispersed in a culture. This makes it possible to stir-culture the CHO cells in the culture so that the CHO cells are not present in a suspended state.

However, this method has problems in that the microcarrier is expensive, and it is difficult to sufficiently increase the cell density in the culture since the cells are present only on the surface of the microcarrier.

For example, Patent Literature 1 discloses a method that cultures CHO cells (i.e., adherent cells) in a suspended state, wherein a gene that encodes the desired protein and a plasmid that has a dihydrofolate reductase gene are transferred into CHO cells, and the resulting recombinant CHO cells are repeatedly cultured at a cell density lower than a normal cell density (i.e., the recombinant CHO cells are cultured in a suspended state).

However, this method has a problem in that repeated culture that causes the CHO cells to be in a suspended state takes time (about 8 weeks). Moreover, it takes more time and cost to manage and check the culture state (e.g., gene mutation and cell alteration) as the subculture period increases.

Patent Literature 2 discloses a method for producing human antithrombin, wherein recombinant CHO cells in a suspended state obtained by transferring a human antithrombin gene and a dihydrofolate reductase gene into dihydrofolate reductase gene-deficient cells, are cultured in a suspended state using a hollow fiber-type culture apparatus to produce human antithrombin.

However, this method has a problem in terms of cost since the hollow fiber-type culture apparatus is complex and expensive. Moreover, when the hollow fiber size is increased to implement mass culture, the culture medium easily becomes non-uniform since the replacement of the medium is non-uniform at each end of the hollow fiber. As a result, the pH and the like of the medium change, and stress is applied to the cells within the hollow fibers, whereby the productivity of the desired protein may decrease.

### CITATION LIST

### PATENT LITERATURE

Patent Literature 1: JP-A-2-009388
Patent Literature 2: JP-A-2005-073509

### NON-PATENT LITERATURE

Non-Patent Literature 1: http://www.gelifesciences.co.jp/catalog/0830.html

### SUMMARY OF INVENTION

### TECHNICAL PROBLEM

As described above, several techniques have been proposed to culture adherent cells at a high density. However, a method that can more efficiently and inexpensively culture adherent cells is desired.

The invention was conceived in view of the above situation. An object of the invention is to provide a method for culturing adherent cells that can allow adherent cells to survive and grow even in a suspended state without requiring a special operation, a culture vessel that is formed using an alicyclic structure-containing polymer, and includes a liquid medium, and adherent cells that survive in the liquid medium in a suspended state, and a method for producing a protein that utilizes the method for culturing adherent cells.

### SOLUTION TO PROBLEM

The inventors conducted extensive studies in order to solve the above problem. As a result, the inventors found that it is possible to allow adherent cells that originally require an extracellular matrix (i.e., scaffold) to survive and grow in a liquid medium in a suspended state by bringing an alicyclic structure-containing polymer formed article into contact with the cultured cells. This finding has led to the completion of the invention.

The present invention provides the following method for culturing adherent cells (see (1) to (5)), culture vessel (see (6) and (7)), and method for producing a protein (see (8)).
(1) A method for culturing adherent cells including bringing adherent cells into contact with an alicyclic structure-containing polymer formed article to grow the adherent cells in a state in which the adherent cells are suspended in a liquid medium.
(2) The method for culturing adherent cells according to (1), wherein the adherent cells are genetically engineered cells that express a foreign gene.
(3) The method for culturing adherent cells according to (1) or (2), wherein the adherent cells are CHO cells.
(4) The method for culturing adherent cells according to (1) or (2), wherein the adherent cells are cells that can express a foreign gene.
(5) The method for culturing adherent cells according to any one of (1) to (4), wherein the adherent cells that are suspended in the liquid medium form a cell mass.
(6) A culture vessel that is formed using an alicyclic structure-containing polymer, the culture vessel including a liquid medium, and adherent cells that survive in the liquid medium in a suspended state.
(7) The culture vessel according to (6), wherein the adherent cells form a cell mass.
(8) A method for producing a protein including bringing recombinant cells that can express a foreign gene that encodes a physiologically active protein into contact with an alicyclic structure-containing polymer formed article when the recombinant cells are cultured.

### ADVANTAGEOUS EFFECTS OF INVENTION

Several aspects of the invention thus provide a method for culturing adherent cells that can allow adherent cells to survive and grow even in a suspended state without requiring a special operation, a culture vessel that is formed using an alicyclic structure-containing polymer, and includes a liquid medium, and adherent cells that survive in the liquid medium in a suspended state, and a method for producing a protein that utilizes the method for culturing adherent cells.

### BRIEF DESCRIPTION OF DRAWINGS

FIG. 1 is a graph illustrating the viable cell count with respect to the number of days elapsed when recombinant CHO cells were cultured.
FIG. 2 is a graph illustrating the concentration of EPO in a culture medium when recombinant CHO cells were cultured for 17 days.
FIG. 3 is a graph illustrating the correlation between the concentration of EPO in a culture medium and the LDH activity value (that reflects the leakage of intracellular components) when recombinant CHO cells were cultured.

### DESCRIPTION OF EMBODIMENTS

A method for culturing adherent cells according to the invention includes bringing adherent cells into contact with an alicyclic structure-containing polymer formed article to grow the adherent cells in a state in which the adherent cells are suspended in a liquid medium.

The adherent cells used in connection with the invention are not particularly limited. The adherent cells used in connection with the invention may be arbitrarily selected corresponding to the object (intended use). The adherent cells used in connection with the invention may be either adherent cells or cells derived from adherent cells. The term "adherent cells" used herein refers to cells that adhere to an extracellular matrix under normal culture conditions to survive and grow. The adherent cells are also referred to as "anchorage-dependent cell". The term "cells derived from adherent cells" used herein refers to cells that are derived from adherent cells and can survive and grow without adhering to an extracellular matrix due to an external factor applied to the adherent cells (e.g., cells obtained by culturing (acclimating) adherent cells so as to be able to survive and grow even in a suspended state). Examples of the adherent cells include host cells used for gene manipulation and virus-sensitive cells, such as CHO cells, VERO cells, NIH3T3 cells, and HEK293 cells. Among these, CHO cells are preferable.

It is preferable that the adherent cells be genetically engineered cells that express a foreign gene. Examples of such cells include cells that can express a foreign gene through transduction using a vector (e.g., phage and plasmid), for example.

The foreign gene may be arbitrarily selected corresponding to the object (intended use). Specific examples of the foreign gene include a gene that encodes a physiologically active protein (e.g., cytokine and hormone) such as erythropoietin (EPO), interferon (interferon α, interferon β, and interferon γ), granulocyte-colony stimulating factor (G-CSF), interleukin, granulocytic-macrophage colony-stimulating factor (GM-CSF), human-growth hormone, insulin, glucagon (HGF), blood coagulation factor VIII, and a human antibody.

When the alicyclic structure-containing polymer formed article is brought into contact with a culture of such genetically engineered cells, the production volume of the physiologically active protein increases.

A liquid medium is used when culturing the cells.

A liquid medium that has a pH buffer action, has an osmotic pressure suitable for the cells, includes nutritional ingredients for the cells, and does not have toxicity to the cells, is normally used as the liquid medium.

Examples of a component that has a pH buffer action include Tris hydrochloride, a phosphate, a carbonate, and the like.

The osmotic pressure of the liquid medium is normally adjusted using an aqueous solution that includes potassium ions, sodium ions, calcium ions, glucose, and the like at an adjusted concentration so that the osmotic pressure of the liquid medium is almost equal to that of the cells. Specific examples of such an aqueous solution include physiological saline such as phosphate buffered saline, Tris-buffered saline, and HEPES-buffered saline; a Ringer's solution such as Ringer's lactate solution, Ringer's acetate solution, and Ringer's bicarbonate solution; and the like.

Examples of the nutritional ingredients for the cells include an amino acid, nucleic acid, a vitamin, a mineral, and the like.

A commercially-available product such as RPMI-1640, HAM, α-MEM, DMEM, EMEM, F-12, F-10, and M-199 may be used as the liquid medium.

An additive may be added to the liquid medium. Examples of the additive include an inducer such as a protein, a low-molecular-weight compound having differentiation-inducing activity, a mineral, a metal, a vitamin component, and the like.

Examples of the additive include a ligand, an agonist, and an antagonist that act on a cell surface acceptor; a ligand, an agonist, and an antagonist that act on a nuclear receptor; an extracellular matrix such as collagen and fibronectin; part of the extracellular matrix, and a compound that imitates the extracellular matrix; a component that acts on a protein that is involved in a cell signaling pathway; a component that acts on a primary or secondary metabolism enzyme within the cells; a component that affects intranuclear or intramitochondrial gene expression; DNA and RNA that can be introduced into the cells in combination with a virus vector or the like; and the like.

These additives may be used either alone or in combination.

The cell culture conditions are not particularly limited. The cell culture conditions may be appropriately determined corresponding to the cells and the object.

For example, the cells may be cultured using a humidified thermostat that contains carbon dioxide at a concentration of about 5%, and is maintained at a constant temperature within the range from 20°C to 37°C.

The alicyclic structure-containing polymer formed article used in connection with the invention is obtained by forming an alicyclic structure-containing polymer so as to have an arbitrary shape.

The term "alicyclic structure-containing polymer" used herein refers to a resin that includes an alicyclic structure in either or both of the main chain and the side chain. It is preferable that the alicyclic structure-containing polymer include an alicyclic structure in the main chain from viewpoint of mechanical strength, heat resistance, and the like.

Examples of the alicyclic structure include a saturated cyclic hydrocarbon (cycloalkane) structure, an unsaturated cyclic hydrocarbon (cycloalkene) structure, and the like. It is preferable that the alicyclic structure-containing polymer include a cycloalkane structure or a cycloalkene structure from the viewpoint of mechanical strength, heat resistance, and the like. It is most preferable that the alicyclic structure-containing polymer include a cycloalkane structure.

The number of carbon atoms included in the alicyclic structure is not particularly limited, but is normally 4 to 30, preferably 5 to 20, and more preferably 5 to 15. When the number of carbon atoms included in the alicyclic structure is within the above range, the alicyclic structure-containing polymer exhibits mechanical strength, heat resistance, and formability in a highly balanced manner.

The content of an alicyclic structure-containing repeating unit in the alicyclic structure-containing polymer may be appropriately selected taking account of the intended use, but is normally 30 wt% or more, preferably 50 wt% or more, and still more preferably 70 wt% or more. If the content of the alicyclic structure-containing repeating unit in the alicyclic structure-containing polymer is too low, the alicyclic structure-containing polymer may exhibit poor heat resistance. A repeating unit that may be included in the alicyclic structure-containing polymer in addition to the alicyclic structure-containing repeating unit is not particularly limited, and may be appropriately selected taking account of the intended use.

Specific examples of the alicyclic structure-containing polymer include (1) a norbornene-based polymer, (2) a monocyclic cycloolefin-based polymer, (3) a cyclic conjugated diene-based polymer, (4) a vinyl alicyclic hydrocarbon-based polymer, hydrogenated products of the polymers (1) to (4), and the like. Among these, a norbornene-based polymer and a hydrogenated product thereof are preferable from the viewpoint of heat resistance, mechanical strength, and the like.

### (1) Norbornene-based polymer

The term "norbornene-based polymer" used herein refers to a polymer that is obtained by polymerizing a norbornene-based monomer (i.e., a monomer that includes a norbornene skeleton). Norbornene-based polymers are roughly classified into a norbornene-based polymer obtained by ring-opening polymerization, and a norbornene-based polymer obtained by addition polymerization.

Examples of the norbornene-based polymer obtained by ring-opening polymerization include a ring-opening polymer of a norbornene-based monomer, a ring-opening polymer of a norbornene-based monomer and a monomer that can undergo ring-opening copolymerization with the norbornene-based monomer, hydrogenated products thereof, and the like. Examples of the norbornene-based polymer obtained by addition polymerization include an addition polymer of a norbomene-based monomer, an addition polymer of a norbornene-based monomer and a monomer that can undergo addition copolymerization with the norbornene-based monomer, and the like. Among these, a hydrogenated ring-opening polymer of a norbornene-based monomer is preferable from the viewpoint of heat resistance, mechanical strength, and the like.

Examples of the norbornene-based monomer include a bicyclic norbornene-based monomer such as bicyclo[2.2.1]hept-2-ene (trivial name: norbornene), 5-methylbicyclo[2.2.1]hept-2-ene, 5,5-dimethylbicyclo[2.2.1]hept-2-ene, 5-ethylbicyclo[2.2.1]hept-2-ene, 5-ethylidenebicyclo[2.2.1]hept-2-ene, 5-vinylbicyclo[2.2.1]hept-2-ene, 5-propenylbicyclo[2.2.1]hept-2-ene, 5-methoxycarbonylbicyclo[2.2.1]hept-2-ene, 5-cyanobicyclo[2.2.1]hept-2-ene, and 5-methyl-5-methoxycarbonylbicyclo[2.2.1]hept-2-ene; a tricyclic norbornene-based monomer such as tricyclo[4.3.0^{1,6}.1^{2,5}]deca-3,7-diene (trivial name: dicyclopentadiene), 2-methyldicyclopentadiene, 2,3-dimethyldicyclopentadiene, and 2,3-dihydroxydicyclopentadiene; a tetracyclic norbornene-based monomer such as tetracyclo[4.4.0.1^{2,5}. 1^{7,10}]-3-dodecene (tetracyclododecene), tetracyclo[4.4.0.1^{2,5}.1^{7,10}]-3-dodecene, 8-methyltetracyclo[4.4.0.1^{2,5}.1^{7,10}]-3-dodecene, 8-ethyltetracyclo[4.4.0.1^{2,5}.1^{7,10}]-3-dodecene, 8-ethylidenetetracyclo[4.4.0.1^{2,5}.1^{7,10}]-3-dodecene, 8,9-dimethyltetracyclo[4.4.0.1^{2,5}.1^{7,10}]-3-dodecene, 8-ethyl-9-methyltetracyclo[4.4.0.1^{2,5}.1^{7,10}]-3-dodecene, 8-ethylidene-9-methyltetracyclo[4.4.0.1^{2,5}.1^{7,10}]-3-dodecene, 8-methyl-8-carboxymethyltetracyclo[4.4.0.1^{2,5}.1^{7,10}]-3-dodecene, 7,8-benzotricyclo[4.3.0.1^{2,5}]dec-3-ene (trivial name: methanotetrahydrofluorene (also referred to as 1,4-methano-1,4,4a,9a-tetrahydrofluorene)), 1,4-methano-8-methyl-1,4,4a,9a-tetrahydrofluorene, 1,4-methano-8-chloro-1,4,4a,9a-tetrahydrofluorene, and 1,4-methano-8-bromo-1,4,4a,9a-tetrahydrofluorene; and the like.

Examples of the monomer that can undergo ring-opening copolymerization with the norbornene-based monomer include a monocyclic cycloolefin-based monomer such as cyclohexene, cycloheptene, cyclooctene, 1,4-cyclohexadiene, 1,5-cyclooctadiene, 1,5-cyclodecadiene, 1,5,9-cyclododecatriene, and 1,5,9,13-cyclohexadecatetraene.

These monomers may be substituted with one substituent, or may be substituted with two or more substituents. Examples of the substituent include an alkyl group, an alkylene group, an aryl group, a silyl group, an alkoxycarbonyl group, an alkylidene group, and the like.

Examples of the monomer that can undergo addition copolymerization with the norbornene-based monomer include an α-olefin-based monomer having 2 to 20 carbon atoms, such as ethylene, propylene, 1-butene, 1-pentene, and 1-hexene; a cycloolefin-based monomer such as cyclobutene, cyclopentene, cyclohexene, cyclooctene, and tetracyclo[9.2.1.0^{2,10}.0^{3,8}]tetradeca-3,5,7,12-tetraene (also referred to as "3a,5,6,7a-tetrahydro-4,7-methano-1H-indene); a non-conjugated diene-based monomer such as 1,4-hexadiene, 4-methyl-1,4-hexadiene, 5-methyl-1,4-hexadiene, and 1,7-octadiene; and the like.

Among these, an α-olefin-based monomer is preferable, and ethylene is more preferable.

These monomers may be substituted with one substituent, or may be substituted with two or more substituents. Examples of the substituent include an alkyl group, an alkylene group, an aryl group, a silyl group, an alkoxycarbonyl group, an alkylidene group, and the like.

A ring-opening polymer of a norbornene-based monomer, or a ring-opening polymer of a norbornene-based monomer and a monomer that can undergo ring-opening copolymerization with the norbornene-based monomer, may be obtained by polymerizing the monomer component in the presence of a known ring-opening polymerization catalyst. Examples of the ring-opening polymerization catalyst include a catalyst that includes a halide of a metal (e.g., ruthenium or osmium), a nitrate or an acetylacetone compound, and a reducing agent, and a catalyst that includes a halide or an acetylacetone compound of a metal (e.g., titanium, zirconium, tungsten, or molybdenum), and an organoaluminum compound.

A hydrogenated ring-opening polymer of a norbornene-based monomer may normally be obtained by adding a known hydrogenation catalyst that includes a transition metal (e.g., nickel or palladium) to a solution including the ring-opening polymer, and hydrogenating the carbon-carbon unsaturated bonds of the ring-opening polymer.

An addition polymer of a norbornene-based monomer, or an addition polymer of a norbornene-based monomer and a monomer that can undergo addition copolymerization with the norbornene-based monomer, may be obtained by polymerizing the monomer component in the presence of a known addition polymerization catalyst. Examples of the addition polymerization catalyst include a catalyst that includes a titanium, zirconium, or vanadium compound and an organoaluminum compound.

### (2) Monocyclic cycloolefin-based polymer

Examples of the monocyclic cycloolefin-based polymer include an addition polymer of a monocyclic cycloolefin-based monomer (e.g., cyclohexene, cycloheptene, or cyclooctene).

### (3) Cyclic conjugated diene-based polymer

Examples of the cyclic conjugated diene-based polymer include a 1,2-addition polymer or a 1,4-addition polymer of a cyclic conjugated diene-based monomer (e.g., cyclopentadiene or cyclohexadiene), a hydrogenated product thereof, and the like.

### (4) Vinyl alicyclic hydrocarbon polymer

Examples of the vinyl alicyclic hydrocarbon polymer include a polymer of a vinyl alicyclic hydrocarbon-based monomer (e.g., vinylcyclohexene or vinylcyclohexane), and a hydrogenated product thereof; a hydrogenated product obtained by hydrogenating the aromatic ring of a polymer of a vinyl aromatic-based monomer (e.g., styrene or α-methylstyrene); and the like. The vinyl alicyclic hydrocarbon polymer may be a copolymer of the above monomer and an additional monomer that is copolymerizable with the above monomer.

The molecular weight of the alicyclic structure-containing polymer is not particularly limited. The polystyrene-equivalent weight average molecular weight of the alicyclic structure-containing polymer determined by gel permeation chromatography using a cyclohexane solution (or a toluene solution when the polymer does not dissolve in cyclohexane) is normally 5,000 or more, preferably 5,000 to 500,000, more preferably 8,000 to 200,000, and particularly preferably 10,000 to 100,000. When the weight average molecular weight of the alicyclic structure-containing polymer is within the above range, the alicyclic structure-containing polymer exhibits mechanical strength and formability in a highly balanced manner.

The glass transition temperature of the alicyclic structure-containing polymer may be appropriately selected taking account of the intended use. The glass transition temperature of the alicyclic structure-containing polymer is normally 50 to 300°C, preferably 100 to 280°C, more preferably 115 to 250°C, and still more preferably 130 to 200°C. When the glass transition temperature of the alicyclic structure-containing polymer is within the above range, the alicyclic structure-containing polymer exhibits heat resistance and formability in a highly balanced manner.

Note that the glass transition temperature of the alicyclic structure-containing polymer refers to a value measured in accordance with JIS K 7121.

The alicyclic structure-containing polymers described above may be used either alone or in combination.

An additive that is normally used for a thermoplastic resin material, such as a soft polymer, an antioxidant, a UV absorber, a light stabilizer, a near-infrared absorber, a release agent, a coloring agent (e.g., dye and pigment), a plasticizer, an antistatic agent, and a fluorescent whitening agent, may be added to the alicyclic structure-containing polymer in an amount that is normally employed.

A polymer other than the soft polymer (hereinafter referred to as "additional polymer") may be mixed with the alicyclic structure-containing polymer. The additional polymer is normally mixed with the alicyclic structure-containing polymer in a ratio of 200 parts by weight or less, preferably 150 parts by weight or less, and more preferably 100 parts by weight or less, based on 100 parts by weight of the alicyclic structure-containing polymer.

If the ratio of the additive or the additional polymer is too large based on the alicyclic structure-containing polymer, the cells may be suspended to only a small extent. Therefore, it is preferable to add (mix) the additive and the additional polymer within such a range that the properties of the alicyclic structure-containing polymer are not impaired.

The additive and the additional polymer may be mixed with the alicyclic structure-containing polymer using an arbitrary method as long as the additive and the additional polymer are sufficiently dispersed in the alicyclic structure-containing polymer. The additive and the additional polymer may be added in an arbitrary order. Examples of the mixing method include a method that mixes (kneads) the resin in a molten state using a mixer, a single-screw kneader, a twin-screw kneader, a roll, a Brabender, an extruder, or the like, a method that dissolves the resin in an appropriate solvent to effect dispersion, and removes the solvent using a coagulation method, a casting method, or a direct drying method, and the like.

When the resin is mixed (kneaded) using a twin-screw kneader, the resulting mixture (kneaded product) is normally extruded in the shape of a rod in a molten state, and cut (pelletized) to have an appropriate length using a strand cutter.

A forming method that is used when forming the alicyclic structure-containing polymer may be arbitrarily selected corresponding to the shape of the alicyclic structure-containing polymer formed article that is brought into contact with cells. Examples of the forming method include an injection forming method, an extrusion method, a cast forming method, an inflation forming method, a blow forming method, a vacuum forming method, a press forming method, a compression forming method, a rotational forming method, a calendering method, a roll forming method, a cutting method, a spinning method, and the like. Note that these methods may be used in combination, and a post-treatment such as stretching may optionally be performed after forming.

The shape of the alicyclic structure-containing polymer formed article is not particularly limited. The alicyclic structure-containing polymer formed article may have a plate-like shape, a powdery shape, a particulate shape, a string-like shape, a sheet-like shape, or the like. The surface of the alicyclic structure-containing polymer formed article may be either flat or irregular. The alicyclic structure-containing polymer formed article may be a hollow formed article. A plurality of formed articles that differ in shape may be combined through an adhesive or the like, or may be combined without using an adhesive or the like, to form another formed article.

The alicyclic structure-containing polymer formed article may be a member that forms part or the entirety of a culture vessel (e.g., dish, plate, bag, tube, scaffold, cup, and jar fermenter), a part of a culture apparatus (e.g., stirring blade, stirring bar, baffle, and connection tube), a culture tool used for culture operation (e.g., pipette, stirring device, filter, and cell scraper), and the like, as long as the cells can be brought into contact with the alicyclic structure-containing polymer formed article.

When implementing the invention, it is preferable to sterilize the formed article before bringing the formed article into contact with the cultured cells.

The sterilization method is not particularly limited. The sterilization method may be selected from sterilization methods that are normally employed in the medical field (e.g., a heating method such as a high-pressure steam method and a dry heat method; a radiation method that applies radiation such as γ-rays or an electron beam, and an irradiation method that applies high-frequency waves; a gas method that brings a gas such as ethylene oxide gas (EOG) into contact with the sterilization target; and a filtration method that utilizes a sterilization filter) corresponding to the shape of the formed article and the cells to be cultured. It is preferable to use a gas method since a change in surface state occurs to only a small extent.

The surface of the formed article may also be subjected to a treatment (e.g., plasma treatment, corona discharge treatment, ozone treatment, and UV irradiation treatment) other than the sterilization treatment that is normally used for a culture vessel. Note that it is preferable to omit such a surface treatment, or subject the formed article to a weak surface treatment that ensures that the water contact angle of the bottom surface (i.e., a surface that comes in contact with a culture) of the container during use is ±20% (more preferably ±10%) with respect to the water contact angle of the bottom surface of the container prior to the surface treatment in order to suppress an increase in cost due to the surface treatment operation, prevent a situation in which cleanliness is impaired due to partial decomposition of the surface of the formed article during the surface treatment, and prevent a decrease in the suspension capability of the cells, for example. Note that the term "water contact angle" used herein refers to a value obtained by cutting the bottom surface of the dish using a circle cutter having a diameter of 30 mm to prepare a specimen, measuring the radius r and the height h of a liquid droplet at the center of the specimen and the vertices of a 20×20 mm square formed around the center of the specimen (i.e., at five measurement points) using an automatic contact angle meter ("LCD-400S" manufactured by Kyowa Interface Science Co., Ltd.), and calculating the value θ using the expressions "tanθ1=h/r" and "θ=2arctan(h/r)" (θ/2 method).

The cultured cells may be brought into contact with the alicyclic structure-containing polymer formed article using an arbitrary method corresponding to the shape of the alicyclic structure-containing polymer formed article. For example, the cultured cells may be brought into contact with the alicyclic structure-containing polymer formed article using a method that cultures the cells in a culture medium into which the alicyclic structure-containing polymer formed article is mixed; a method that cultures the cells in a culture vessel that is formed using the alicyclic structure-containing polymer; a method that cultures the cells using a culture tool that is formed using the alicyclic structure-containing polymer; or the like. Note that these methods may be used in combination.

Since cells have a signaling capability, all of the cultured cells need not necessarily come in contact with the alicyclic structure-containing polymer formed article, and the cultured cells need not necessarily come in contact with the alicyclic structure-containing polymer formed article during the entire culture period. Note that it is preferable that the contact time be as long as possible in order to compensate for a decrease in effect with the passing of time.

The temperature at which the cultured cells are brought into contact with the alicyclic structure-containing polymer formed article is not particularly limited as long as the cells can be grown.

The cells that have been cultured for a long time using the method according to the invention and survive in a suspended state normally form a cell mass. The term "cell mass" used herein refers to an aggregate of cells (other than a single cell) (i.e., a state in which two or more cells aggregate to form a mass).

The term "survive" used herein in connection with the cells means that metabolic activity is observed in the cells, and the cells can be grown. For example, the term "survive" used herein in connection with the cells means that, when a component included in the culture medium has entered the cells, and the component is unnecessary for the vital activity of the cells, the cells can eliminate the component from the cells through vital activity. Whether or not the cells survive may be experimentally determined by adding a dye (e.g., trypan blue) that is unnecessary for vital activity to the extracellular liquid, and determining whether or not the cells can eliminate the dye that has entered the cells to the outside (i.e., cells that can eliminate the dye to the outside are determined to be surviving cells, and cells that cannot eliminate the dye to the outside are determined to be dead cells).

When the method according to the invention is used, the cells that form a cell mass can survive, and transduced cells can grow and produce a protein.

Since the method according to the invention can culture the adherent cells in a suspended state, it is possible to culture the adherent cells at high density.

The term "high density" used herein means that the cell count is larger than the confluent cell count of the adherent cells cultured using a polystyrene dish by a factor of 1.5 or more, and preferably 2 or more.

Since the culture method according to the invention can culture the adherent cells in a suspended state at a high density, the culture method according to the invention may preferably be used when causing the cells to produce a protein.

For example, it is possible to produce a large amount of protein by bringing recombinant cells that can express a foreign gene that encodes a physiologically active protein into contact with the alicyclic structure-containing polymer formed article when the recombinant cells are cultured.

### EXAMPLES

The invention is further described below by way of examples. Note that the invention is not limited to the following examples.

### Production Example 1: Production of recombinant EPO-producing CHO cells

The EPO gene sequence was inserted into the insertion site of the expression gene of a vector pLXRN (manufactured by Chlontech) including a gene resistant to an antibiotic G418 to obtain a plasmid pLXRN-EPO. The presence of the EPO gene in the plasmid was determined by analyzing the base sequence.

The plasmid pLXRN-EPO and a plasmid pVSV-G (manufactured by Clontech) were transduced into GP293T cells (manufactured by Clontech) (packaging cells) to prepare virus particles including the EPO gene.

Note that Lipofectamine (manufactured by Invitorogen) was used as a gene transfection reagent for transfecting the plasmid pLXRN-EPO into the cells, and the gene transfection operation was performed in accordance with the manufacturer's manual.

The GP293T cells subjected to the gene transfection operation were cultured, and the culture supernatant was removed, and filtered through a filter. 8 µg/ml of polybrene (manufactured by Santa Cruz) was added to the filtrate to prepare a culture supernatant sample including the virus particles including the EPO gene.

The culture supernatant sample including the virus particles including the EPO gene was added to a CHO cell sample cultured in advance, and the mixture was cultured for 8 hours to infect the CHO cells with the virus particles including the EPO gene.

After performing incubation for 8 hours, the culture medium was replaced with a CHO cell culture medium, and recombinant EPO-producing CHO cells were cultured.

Infection with the virus was checked by genomic PCR. Specifically, the genome was extracted from the CHO cells subjected to the infection operation using an InstaGene matrix (manufactured by BioRad) to determine that the pLXRN-EPO sequence was introduced into the genome by PCR. Primers pLXRN-seq-F (5'-CGCCTCCGTCTGAATTTTT) and pLXRN-seq-R (TCCCTATGCAAAAGCGAAAC) were used for PCR.

In order to select the CHO cells which were infected with the virus and in which the EPO gene was introduced into the genome, an antibiotic G418 was added to the culture medium, and the mixture was cultured. The CHO cells resistant to the antibiotic G418 were selected using a reagent to select recombinant EPO-producing CHO cells (hereinafter referred to as "EPO-producing CHO cells").

### Example 1

A dish having a diameter of 3 cm was produced by an injection forming method using an alicyclic structure-containing polymer "ZEONEX (registered trademark) 790R" (manufactured by Zeon Corporation, hydrogenated norbornene-based ring-opening polymer, hereinafter referred to as "790R"). The resulting dish is hereinafter referred to as "790R dish".

The EPO-producing CHO cells were seeded onto a Ham's medium (liquid medium) containing 10% fetal bovine serum at a cell density of 1.25×10⁴ cells/cm² using the 790R dish as a culture vessel, and cultured at 37°C for 17 days in a 5% CO₂ atmosphere. On the 11th day from the start of culture, it was observed that the volume of the culture medium had decreased due to transpiration of water, and the culture medium was added to the 790R dish in the same volume as the volume that had decreased.

The viable cell count was measured as described below on the 2nd day, 7th day, 10th day, 14th day, and 17th day from the start of culture.

### Measurement of cell count

With regard to the cells in a suspended state, the culture supernatant was removed, and centrifuged to effect precipitation. The cells were collected, and stained with trypan blue in order to distinguish the viable cells and the dead cells, and the viable cell count was measured.

With regard to the cells adhering to the bottom surface of the dish, the cells were washed with physiological saline, removed from the dish by trypsinization, and stained with trypan blue in order to distinguish the viable cells and the dead cells, and the viable cell count was measured.

### Comparative Example 1

Cells were cultured in the same manner as in Example 1, except that a polystyrene dish ("Falcon (registered trademark) 353001" manufactured by Becton, Dickinson and Company) (hereinafter referred to as "polystyrene dish") was used instead of the 790R dish, and the viable cell count was measured in the same manner as in Example 1.

When the EPO-producing CHO cells were cultured using the 790R dish, it was observed on the 7th day from the start of culture that the EPO-producing CHO cells formed a mass, and had grown in a non-contact state. When the EPO-producing CHO cells were cultured using the polystyrene dish, most of the surviving EPO-producing CHO cells adhered to the bottom surface of the polystyrene dish. The EPO-producing CHO cells suspended in the culture medium were present alone, and most of them were dead.

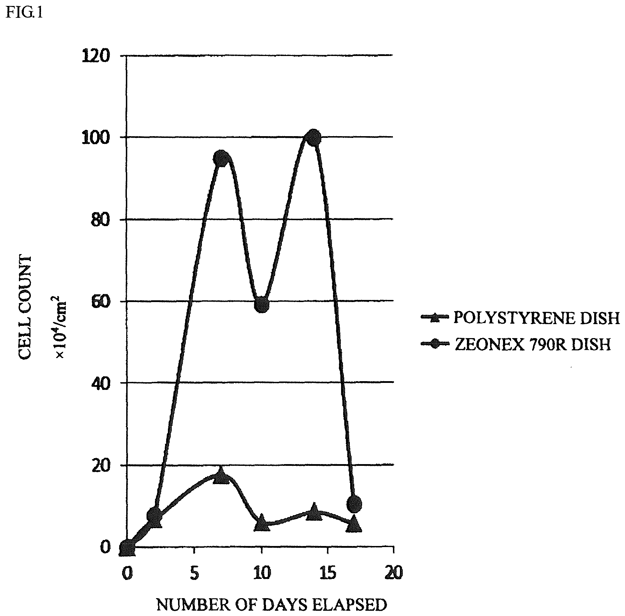

As illustrated in FIG. 1, the viable cell count on the 7th day from the start of culture achieved using the 790R dish was larger than that achieved using the polystyrene dish by a factor of about 5.

A decrease in viable cell count was observed on the 10th day from the start of culture with respect to both the 790R dish and the polystyrene dish. However, an increase in viable cell count was observed on the 11th day from the start of culture (on which the culture medium was added) when the 790R dish was used. It was thus confirmed that the 790R dish makes it possible to easily increase the viable cell count through the addition of the culture medium, for example.

When the polystyrene dish was used, a significant increase in viable cell count was not observed even when the culture medium was added.

### Example 2

The EPO-producing CHO cells were repeatedly cultured three times using the 790R dish as a culture vessel. The amount of active EPO was measured by ELISA ("human EPO Platinum ELISA" manufactured by eBioscience) using the culture medium on the 17th day after the start of culture.

### Comparative Example 2

Cells were cultured in the same manner as in Example 2, except that the polystyrene dish was used instead of the 790R dish, and the amount of active EPO was measured in the same manner as in Example 2.

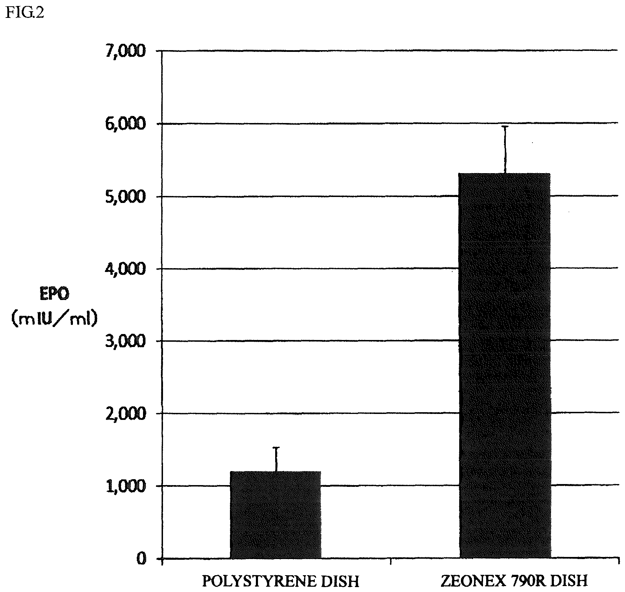

As illustrated in FIG. 2, the amount of EPO produced from the EPO-producing CHO cells cultured using the 790R dish was larger than the amount of EPO produced from the EPO-producing CHO cells cultured using the polystyrene dish (that corresponds to existing culture technology) by a factor of about 5. It was thus confirmed that the invention makes it possible to produce EPO at a high concentration that cannot be achieved using known technology.

### Example 3

The EPO-producing CHO cells were cultured using the 790R dish as a culture vessel. The activity of lactate dehydrogenase (LDH) (intracellular metabolic enzyme) was measured using the culture medium to determine the degree of leakage of intracellular components due to the death of the EPO-producing CHO cells. Note that the activity of LDH was measured using an LDH Cytotoxicity Detection Kit (manufactured by Takara Bio Inc.).

### Comparative Example 3

Cells were cultured in the same manner as in Example 3, except that the polystyrene dish was used instead of the 790R dish, and the activity of LDH was measured in the same manner as in Example 3.

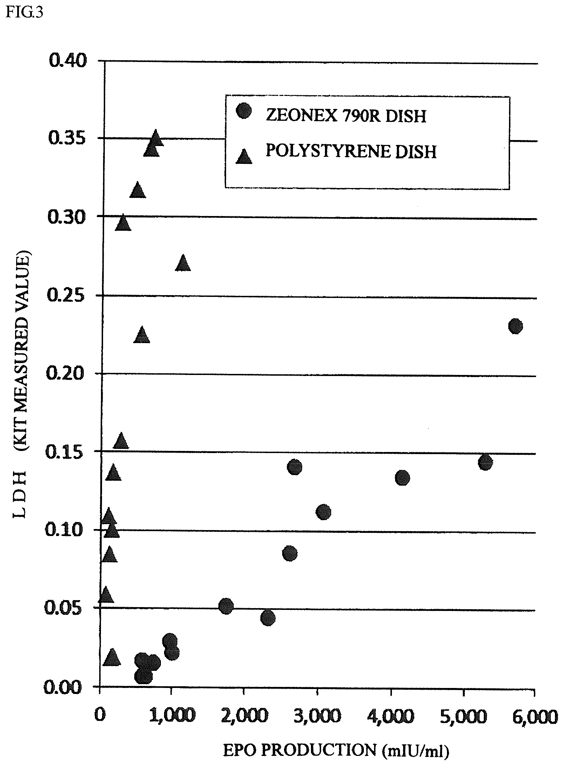

In the graph illustrated in FIG. 3, the horizontal axis indicates the EPO concentration in the culture medium, and the vertical axis indicates the LDH activity value. The measured values from the culture medium when the cells were cultured using the 790R dish are indicated by circles, and the measured values from the culture medium when the cells were cultured using the polystyrene dish are indicated by triangles.

When the 790R dish and the polystyrene dish were used, the amount of leakage components from the cells increased as the EPO concentration increased. The amount of leakage components from the cells when the cells were cultured using the 790R dish was smaller than that when the cells were cultured using the polystyrene dish.

### INDUSTRIAL APPLICABILITY

The method according to the invention can culture cells at a higher density when producing a recombinant protein using cells, and obtain the desired protein at a high concentration. Therefore, it is possible to reduce the number of operations during cell culture, reduce the culture scale, and reduce the labor cost, device cost, culture medium cost, and the like.

Since the desired protein can be obtained at a high concentration, it is possible to easily concentrate the protein that is easily denatured, and reduce the risk that the protein will be denatured.

Since the amount of leakage components from the cells is small, it is possible to reduce the purification cost when purifying the protein (i.e., it is economically advantageous). Since it is considered that a protein with higher purity can be obtained through purification, it is possible to reduce the risk that a component that may cause side effects (e.g., fever) will be mixed.

In the field of biotechnological research such as drug development research, it is considered that a high-purity recombinant protein can be obtained at a high concentration on a small scale by utilizing the method according to the invention. Therefore, the method according to the invention can contribute to the smooth progress of physiological activity evaluation experiments and the like. This makes it possible to reduce the development cost and the production cost of drugs and the like.

## Claims

1. A method for culturing adherent cells comprising bringing adherent cells into contact with an alicyclic structure-containing polymer formed article to grow the adherent cells in a state in which the adherent cells are suspended in a liquid medium.

2. The method for culturing adherent cells according to claim 1, wherein the adherent cells are genetically engineered cells that express a foreign gene.

3. The method for culturing adherent cells according to claim 1 or 2, wherein the adherent cells are CHO cells.

4. The method for culturing adherent cells according to claim 1 or 2, wherein the adherent cells are cells that can express a foreign gene.

5. The method for culturing adherent cells according to any one of claims 1 to 4, wherein the adherent cells that are suspended in the liquid medium form a cell mass.

6. A culture vessel that is formed using an alicyclic structure-containing polymer, the culture vessel comprising a liquid medium, and adherent cells that survive in the liquid medium in a suspended state.

7. The culture vessel according to claim 5, wherein the adherent cells form a cell mass.

8. A method for producing a protein comprising bringing recombinant cells that can express a foreign gene that encodes a physiologically active protein into contact with an alicyclic structure-containing polymer formed article when the recombinant cells are cultured.
